# EUROPEAN PATENT SPECIFICATION

(11) **EP 1 023 915 B1**
(45) Date of publication and mention of the grant of the patent: **02.01.2004**
(21) Application number: 00109150.3
(22) Date of filing: 18.03.1998
(51) Int. Cl.: A61N 1/05

(54) **Medical electrical lead**
Leitung für medizinische Zwecke
Conducteur électrique médical

(30) Priority: 21.04.1997 US 843763; 21.04.1997 US 843765
(43) Date of publication of application: 02.08.2000
(62) Divisional of application: 98911775.9
(73) Proprietor: MEDTRONIC, INC., Minneapolis, Minnesota 55432-3576 (US)
(72) Inventor: Verness, David D., Forest Lake, Minnesota 55025 (US); Huepenbecker, George M., Vadnais Heights, Minnesota 55110 (US); Wahlstrom, Dale A., Plymouth, Minnesota 55442 (US); Swoyer, John M., Andover, Minnesota 55304 (US); Hebzynski, Annette, Mesa, Arizona 58208 (US)
(74) Representative: Hughes, Andrea Michelle

(56) References cited:
- EP-A- 0 574 358
- EP-A- 0 672 431
- EP-A- 0 672 432
- DE-A- 3 640 033
- US-A- 4 033 355
- US-A- 5 231 996
- US-A- 5 324 321

## Description

The present invention relates to implantable electrical leads generally, and more specifically to cardiac pacing leads.

The conductors in cardiac pacing leads occasionally have a tendency to fracture due to repetitive application of stress to the conductor. One way in which this problem has previously been addressed is by reinforcing the lead body in the area in which stress is to be expected, as in U.S. Patent No. 5,545,203, issued to Doan et al. This patent is directed primarily toward reinforcing the lead against fracture due to application of compressive forces. Reinforcement of the lead body is also disclosed in U.S. Patent No. 5,591,142, issued to Van Erp et al.. It has also been proposed to reinforce the lead body by means of adding a tensile reinforcement as in U.S. Patent No. 5,231,996 issued to Bardy et al. In this patent, the lead is provided with a non-conductive tensile member such as a polyester cord, which runs the length of the lead body. Other leads having cords or reinforcements running throughout their length are disclosed in U.S. Patent No. 3,844,292 and U.S. Patent No. 3,572,344 issued to Bolduc. A third proposal for dealing with the possibility of conductor fracture is to render the portion of the lead body in direct contact with the conductor conductive by addition of carbon or other conductive material, as disclosed in U.S. Patent No. 4,033,355, issued to Ammundson.

EP-A-0672431 discloses a safety element for assuring the electrical continuity of pacemaker leads, comprising a flexible lead formed by one or more wires coiled in tight turns or intertwined for introduction into the bore of the main metal coil of the pulse transmitting lead.

The present invention is directed toward providing a lead which has an increased resistance to fracture and has the capability of continued function after fracture of a conductor. The lead is provided with a coiled conductor which may be monofilar or multifilar and which extends along the length of the lead, running from an electrical connector at the proximal end of the lead to an electrode at or near the distal end of the lead. In addition, the lead is provided with a stranded conductor which extends along the coiled conductor from a point along the lead body located proximal to the point of expected breakage of the coiled conductor to a point along the lead body located distal to the point of expected breakage. The proximal and distal ends of the stranded conductor in some embodiments are electrically and mechanically coupled to the coiled conductor, limiting the extensibility of the coiled conductor, rendering the coiled conductor less susceptible to axially applied tensile forces and also providing for continued electrical connection to the electrode, in the event that the coiled conductor fractures intermediate the proximal and distal ends of the stranded conductor.

Preferred embodiments of the invention will now be described, by way of example only, with reference to the drawings.
Figure 1 is a plan view of an implantable lead in which the present invention is practiced.
Figure 2 is cross-sectional view through the lead of Figure 1, illustrating a second embodiment of the invention.
Figure 3 is a side, cut-away view through the lead of Figure 1, illustrating the second embodiment of the invention.
Figure 4 is a side, cut-away view through the distal portion of the lead of Figure 1, illustrating the second embodiment of the invention.
Figure 5 is a side, cut-away view through the connector assembly of the lead of Figure 1, illustrating the second embodiment of the invention.
Figure 6 is a side, cut-away view through the lead of Figure 1, illustrating a third embodiment of the invention.
Figure 7 is cross-sectional view through the lead of Figure 1, illustrating a fifth embodiment of the invention.
Figure 8 is a side, cut-away view through the lead of Figure 1, illustrating the fifth embodiment of the invention.
Figure 9 is a plan view of a lead having a rotatable fixation helix, embodying the present invention.
Figure 10 shows a plan view of an epicardial lead employing the present invention.
Figure 11 illustrates a sectional view through the lead of Figure 10, at a point intermediate the connector assembly and point B.
Figure 12 illustrates one mechanism for interconnecting the coiled conductor, the stranded conductor and the connector pin in the connector assembly of the lead illustrated in Figure 10.
Figure 13 illustrates the interconnection of the stranded conductor to the coiled conductor at point B, illustrated in Figure 20.

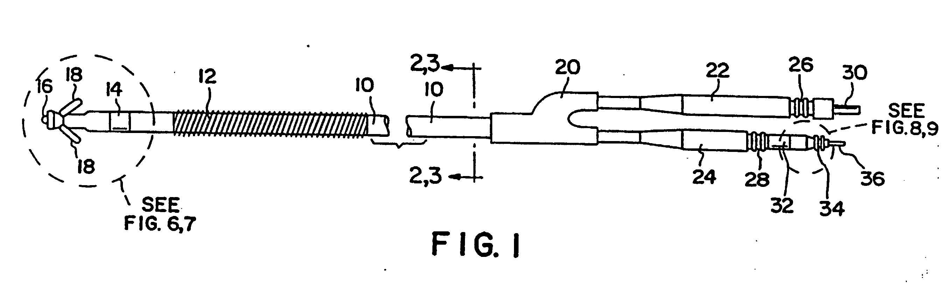

Figure 1 is a plan view of a defibrillation lead of a lead in which the present invention is practiced. The present invention, of course, may also be usefully practiced in the context of other types of medical electrical leads, such as cardiac pacing leads, nerve and muscle stimulation leads, and so forth.

The lead of Figure 1 is provided with an elongate insulative lead body 10, preferably fabricated of silicone rubber, polyurethane or other biocompatible elastomer. At the proximal end of the lead, it carries an elongate defibrillation electrode 12, a ring electrode 14 and a tip electrode 16, each coupled to a conductor located within the lead body 10. Tines 18 are provided in maintaining electrode 16 in contact with the tissue of the right ventricle. Electrodes 16, 14 and 12 may correspond generally to conventionally available pacing and defibrillation electrodes.

The proximal end of the lead carries a connector assembly, beginning with a molded lead bifurcation 20, which splits off two of the conductors within lead body 10 to a bipolar, in-line connector assembly 24, generally corresponding to the IS-1 connector standard for pacing leads. However, other types of connector assemblies may also be adapted to practice the present invention. Connector assembly 24 is provided with a first set of sealing rings 28, a connector ring 32, a second set of sealing rings 34 and connector pin 36. Connector pin 36 is coupled to the conductor which extends through the lead body 10 to tip electrode 16. The connector ring is coupled to the conductor which extends through the lead body 10 to ring electrode 14. The conductor coupled to defibrillation electrode 12 extends into connector assembly 22, which carries a set of sealing rings 26 and a connector pin 36, coupled to the conductor extending through lead body 10 to defibrillation electrode 12.

In the specific context of the lead illustrated in Figure 1, the conductor coupling connector pin 36 to electrode 16 takes the form of a monofilar or multifilar coiled conductor to allow passage of a stylet therethrough, while the conductors coupling ring electrode 14 to connector ring 32 and coupling defibrillation electrode 12 to connector pin 30 take the form of bundled, stranded wires, provided with a coating of PTFE. However, the conductors coupling ring electrode 14 and defibrillation electrode 12 may take the form of any of the various conductor types known for use in conjunction with implantable electrical leads. If fewer electrodes are provided on the lead, correspondingly fewer conductors will be included. One or more physiologic sensors may be added to the lead or substituted for one or more of the illustrated electrodes. Also located within lead body 10 is a stranded wire conductor which extends along a length of the coiled conductor and which serves as a mechanism for bridging a fracture of the coiled conductor which occurs between the ends of the stranded conductor. In some embodiments, the stranded conductor also couples electrode 16 to connector pin 36, providing both an axial reinforcement and a redundant electrical connection, as described in more detail below. In other embodiments, the electrical interconnection between the coiled and stranded conductors may simply be the contact between the two conductors which occurs as a result of both conductors being located in the same lumen of the lead.

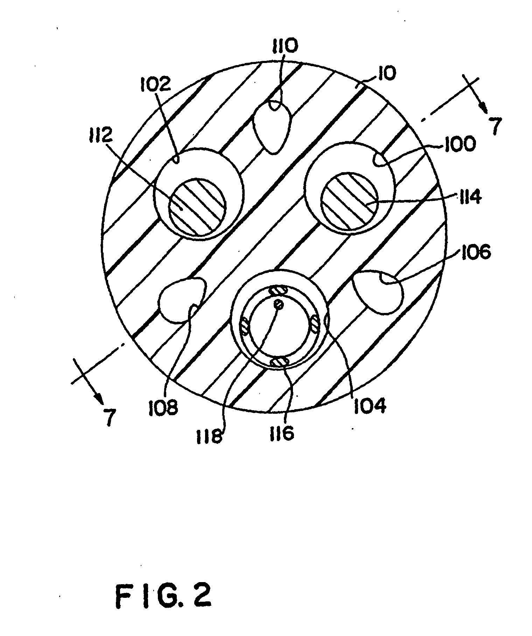

Figure 2 illustrates a cross-section through lead body 10, illustrating the inter-relation of the conductor lumens 100, 102 and 104 with compression lumens 106, 108 and 110, which are described in more detail in U.S. Patent No. 5,584,873, issued to Shoberg et al. In this view it can be seen that lumens 100 and 102 contain conductors 112 and 114 which in the illustrated embodiment may take the form of PTFE coated bundled stranded wires having a generally straight configuration. In particular, conductors 112 and 114 may take the form of a PTFE coated, bundled, stranded 49 filar cable formed of seven strands, each strand formed of seven filars, as described in more detail in U.S. Patent No. 5,584,873 by Shoberg et al. Lumen 104 contains a conventional multifilar coiled conductor 116 and a small diameter bundled stranded wire conductor 118. Conductor 118 may take the form of a seven filar bundle or cable of MP35N or silver cored MP35N wire, as described in U.S. Patent No. 5,246,014, issued to Williams et al, such that conductor 118 corresponds generally to one of the seven strands that make up conductors 112 and 114. In preferred embodiments, conductor 118 may have an outer diameter of about 0.075mm (.003 inches).

In spite of its small diameter and generally straight configuration, stranded conductor 118 is extremely resistant to fracturing in response to repeated flexure of the lead body and displays a high tensile strength. Thus, should coil conductor 116 fracture, redundant, stranded conductor 118 will remain to provide for connection to the electrode to which coiled conductor 116 is coupled. If the stranded and coiled conductors are uninsulated from one another, they make contact with one another at multiple points along the lead body, so that a break of the coiled conductor occurring between the ends of the stranded conductor will be bridged. The ends of conductor 118 may also be mechanically coupled to the coiled conductor 116 and thereby serve to maintain the structural integrity of the lead, preventing partial disassembly due to applied tensile forces. If the lead is removed, conductor 118 may thus also serve as a reinforcement, allowing traction force to be applied to the distal end of the lead during extraction. In either case, conductor 118 allows for continued functioning of the lead after fracture of the coiled conductor 116, allowing for replacement of the lead, when convenient, without interruption of the therapeutic function of the pacemaker or stimulator to which the lead is coupled.

In some embodiments of the invention, conductor 118 is uninsulated along its length and thus makes contact with conductor 116 at various points along the length of the lead. In such embodiments, it is to be expected that the conductor 118 will serve as both a redundant conductor, coupling the connector pin 36 to the electrode 16, and as a conductive bridge between the broken ends of the conductor 116, as it will be in contact with the conductor 116 on either side of the break. With this structure, changes in overall impedance between the connector pin and electrode are expected to be relatively small, allowing for essentially undiminished performance of the lead. Alternatively, conductor 118 may be provided with an insulative coating of PTFE or other insulative material. In such embodiments, conductor 118 will serve as a redundant connector, connecting connector pin 32 to electrode 16, and upon fracture of conductor 116, a substantial change in connector pin to electrode impedance will be manifested. In the context of implantable stimulators capable of monitoring changes in lead impedance, this provides the physician and/or the device itself with a mechanism for detecting the fracture in 116. However, within the context of the present invention, the fracture can be detected without the serious consequences which would otherwise be associated with disconnection of the electrode 16 from the connector pin 36. In the context of implantable stimulators having the ability to automatically adjust stimulus pulse amplitude and input amplifier sensitivity, the device may respond to the change in lead impedance by noting the occurrence of a fracture in conductor 116 and may correspondingly alter its programmed parameters in order to restore performance essentially to that preceding the fracture of conductor 116.

The stranded conductor 118 is located within the lumen of conductor 116. This feature may be particularly advantageous in the context of leads, such as epicardial electrode leads or some nerve and muscle stimulation leads which do not require passage of a stylet through the lumen of coil conductor 116.

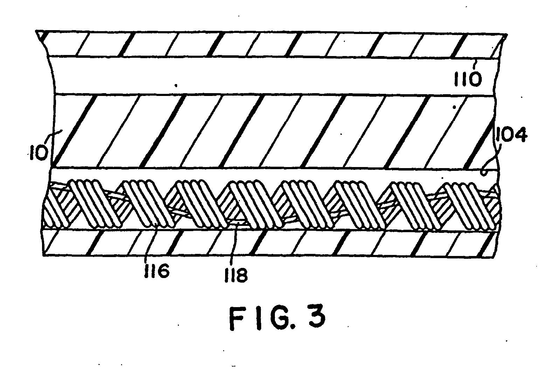

FIG. 3 shows a side cutaway view through the second embodiment of the lead of FIG. 1, also illustrated in FIG. 2. In this view, the stranded conductor is shown arranged loosely within the lumen of coiled conductor 116.

In the embodiments illustrated in Figures 2 and 3, conductor 118 may be insulated or uninsulated, as discussed above, depending on whether contact between the two conductors along their length is desired. An alternative embodiment in which the stranded conductor is desired to be insulated from the coiled conductor along some portion of its length may employ a separate lumen in the lead body for the stranded conductor, intermediate its points of connection to the coiled conductor. An additional alternative as discussed below may employ a tubular, insulative sheath within coiled conductor 116 to insulate it from conductor 118.

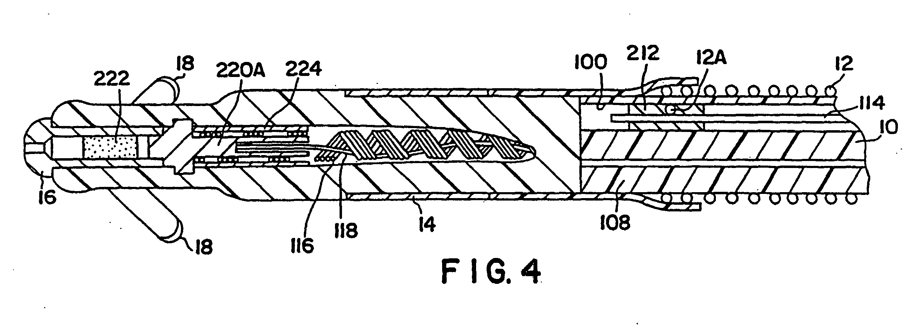

FIGs. 4 et seq. show basic mechanisms which may optionally be employed to mechanically interconnect the stranded conductor 118, the coiled conductor 116, electrode 16 and connector pin 36. These illustrated interconnection mechanisms are intended to be exemplary, and may of course, be employed in conjunction with other components of implantable leads, including other types of electrical connectors such as connector rings, corresponding to connector ring 32 and to interconnect these conductors with other types of electrodes and to interconnect these components with other lead components such as physiologic sensors such as pressure sensors, oxygen sensors, temperature sensors and the like.

Figure 4 is a sectional view through the distal portion of the lead illustrated in Figure 1. In this view, the interconnection of conductor 116, conductor 118 and electrode 16 is visible. Extending distally from the defibrillation electrode 12, the lead takes the form of a molded piece part 228, which carries ring electrode 14, which is in turn coupled to stranded conductor 112 (not visible in this view). Electrode 16 as illustrated is a steroid-eluting electrode, provided with a monolithic controlled release device 222 located within a chamber within the electrode. Electrode 16 is coupled to a coiled conductor 116 and 118 by means of an external crimping sleeve 224, which compresses conductor 116 against the proximal portion 220 of electrode 16. A bore is provided in the proximal section 220A of electrode 16, and stranded conductor 118 is crimped therein. Other types of tip electrodes, including screw-in electrodes may of course be substituted for electrode 16. Similarly, other mechanisms may be employed to interconnect conductors 118 and 116 and electrode 16, including welding, swaging, crimping and combinations thereof, including mechanisms as disclosed in commonly assigned, copending U.S. Patent No. 5,676,694 by Boser et al, filed June 7, 1996 and U.S. Patent No. 6,026,567 by Swoyer et al., filed May 11, 1995.

Conductor 114 passes through an internal lumen 100 within lead body 10, and has its insulation removed in areas in which it passes through the cross-bore crimp sleeve 212. The distal turn of electrode coil 12 can be seen at 12A as it passes through the perpendicular cross-bore through sleeve 212. The sleeve 212 is crimped to the conductor 114 and a portion of the distal turn of electrode coil 12 is inserted through the cross bore and the entry and exit points of the coil are laser welded to the sleeve. External polymeric sleeve 230 is slid over the distal ends of conductor coil 12, and the areas between the sleeve 230 lead body 10 is backfilled by means of medical adhesive or other polymeric material. The electrode coil 12 may be secured to the outer circumference of the lead body 10 by means of a backfilling process as described in U.S. Patent No. 4,934,049.

While Figure 4 shows the inter-connection of the stranded and coiled conductors at the tip electrode 16, these conductors may instead be connected at a point proximal to the tip electrode, for example by use of a cross-bore crimp sleeve similar to sleeve 212, or by means of other types of welded, swaged or crimped connections as discussed above.

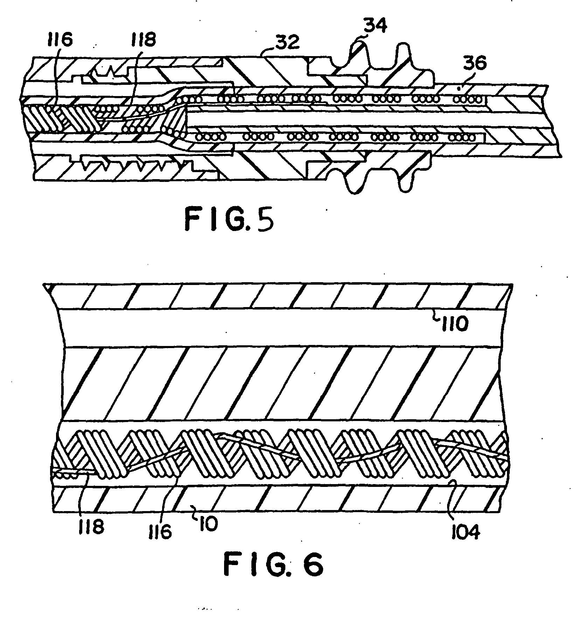

Figure 5 is sectional view through the bipolar connector assembly 24 of the lead illustrated in Figure 1, illustrating the first embodiment of the invention. In this view, the proximal end of connector pin 36 is visible in cross-section, and connector ring 32 is visible in cross-section. Connector pin 36 is coupled to coiled conductor 116 by means of a swaging core 200, which compresses conductor coil 116 and stranded conductor 118 between the interior lumen of connector pin 36 and the outer surface of swaging core 200, in a conventional fashion. An insulative sleeve 206 surrounds conductors 116 and 118, and extends distally, back through the connector assembly into molded sealing ring sleeve 28 (Fig. 1).

Surrounding connector pin 36 is a molded sealing ring sleeve 34, which may be fabricated of silicone rubber, which in turn is mounted to a spacer 204 which is typically fabricated of a harder plastic, such as polyurethane. Spacer 204 is molded in situ between connector pin 36 and ring electrode 32, and is maintained in mechanical interconnection with electrode 32 by means of internal threading 208, as described in U.S. Patent No. 4,572,605, issued to Hess, et al.

As in the case of figure 4 above, other mechanisms may be employed to interconnect conductors 118 and 116 and connector pin 36, including welding, swaging, crimping and combinations thereof, as described above. Additionally, these conductors may instead be connected at a point distal to the connector pin, for example by use of a cross-bore crimp sleeve similar to sleeve 212, or by means of other types of welded, swaged or crimped connections as discussed above.

If it is not desired to mechanically interconnect on or both ends of the stranded conductor 118 to the coiled conductor 116, the internal structure of the leads may correspond to those illustrated in Figures 4 or 5 above, with the exception the stranded conductor 118 is simply not crimped, swaged or otherwise coupled to the connector pin, electrode or coiled conductor 118. In such embodiments, the stranded conductor may extend the entire length of the coiled conductor or may extend over only a portion of the length of the coiled conductor. While the Figures 4 and 5 illustrate the coil and stranded conductor pair coupled to the connector pin and tip electrode, it should also be understood that the invention may also be usefully practiced in leads in which these conductors are coupled to other connector elements, other electrodes and/or physiologic sensors located on the lead body. The interconnection methods of Figures 4 and 5 may also be used to connect the stranded conductor 118 to the coiled conductor 116 and to such other lead components.

Figure 6 illustrates a third embodiment of the invention. All numbered components correspond to identically numbered components in the Figures above. In this embodiment, an uninsulated stranded conductor 118 repeatedly enters and exits the internal lumen of the coiled conductor 116, by passing between the coils. This embodiment, while more difficult to assemble, provides for in increase in the number of contact points between the stranded and coiled conductors, which may be beneficial in the case of coil fractures as it will in many cases shorten the distance which the stranded conductor must bridge as compared to the first and second embodiments and may provide for more consistent contacts between the stranded and coiled conductors.

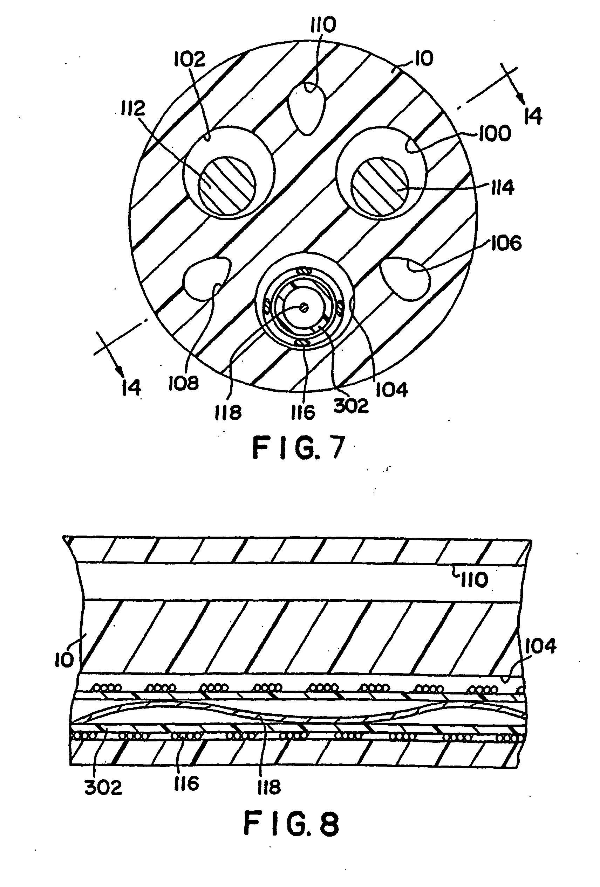

Figure 7 illustrates a cross section through a further embodiment of the invention All numbered components correspond to identically numbered components in the Figures above. In this embodiment the stranded conductor 118 is located inside of coiled conductor 116 and is insulated from conductor 116 over at least a portion of its length by means of an insulative tube 302, located interior to conductor 116. Tube 302 may be formed of PTFE or other insulative biocompatible plastic, and may extend along all or some of the length of coiled conductor 116. In this embodiment, it is desirable that the ends of stranded conductor 118 are mechanically coupled to the coiled conductor 116 on either side of the tube 302.

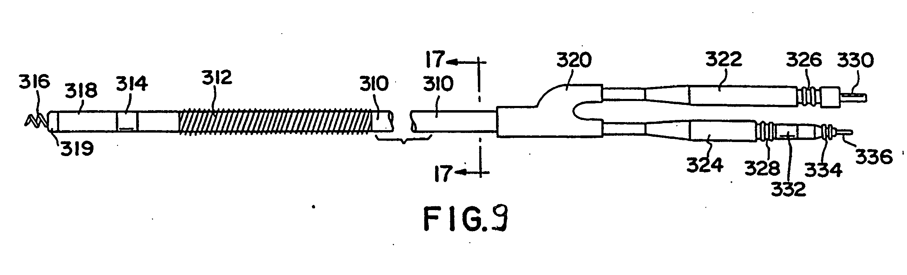

Figure 8 illustrates a side, cut-away view through the fifth embodiment of the invention as illustrated in Figure 7. All numbered components correspond to identically numbered components in the Figures above.

Figure 9 is a plan view of a defibrillation lead in which the present invention is practiced, employing a tip electrode taking the form of a rotatable fixation helix 316. The lead of Figure 9 is provided with an elongated insulative lead body 310, preferably fabricated of silicone rubber, polyurethane or other biocompatible elastomer. At the distal end of the lead, it carries an elongated defibrillation electrode 312, a ring electrode 314 and a rotatable helical tip electrode 316, rotatably and advancably mounted in insulative electrode head 318. Each electrode is coupled to a conductor located within the lead body 310. Electrodes 314 and 312 may correspond generally to conventionally available pacing and defibrillation electrodes. A cap member 319 is located at the distal end of electrode head 318 and serves to retain a monolithic controlled release device as discussed below.

The proximal end of the lead carries a connector assembly, beginning with a molded lead bifurcation 320, which splits off two of the conductors within lead body 310 to a bipolar, in-line connector assembly 324, generally corresponding to the IS-1 connector standard for pacing leads. However, other types of connector assemblies may also be adapted to practice the present invention. Connector assembly 324 is provided with a first set of sealing rings 328, a connector ring 332, a second sealing rings 334 and connector pin 336. Connector pin 336 is rotatably mounted and is coupled to a rotatably mounted conductor which extends through the lead body 310 to helical electrode 316. Connector ring 332 is coupled to a conductor which extends through the lead body 310 to ring electrode 314. A conductor coupled to defibrillation electrode 312 extends into connector assembly 322, which carries a set of sealing rings 326 and is coupled to connector pin 336.

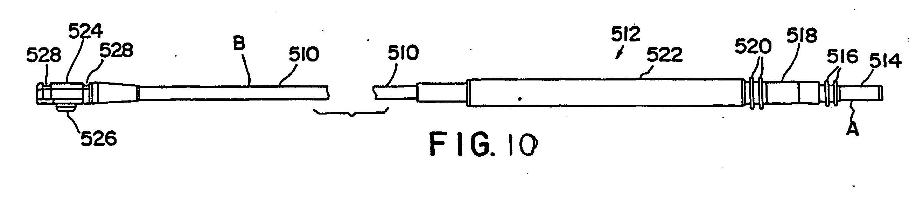

Figure 10 shows a plan view of an epicardial lead employing the present invention. As illustrated, the lead is provided with an insulative elongated lead body 510 which carries a connector assembly 512 at its proximal end. The overall external configuration of the connector assembly as illustrated corresponds to the international "IS-1" connector standard, presently employed in conjunction with implantable cardiac pacing leads. At the proximal end of the connector assembly 512 is a connector pin 14 which is coupled by means of a coiled conductor extending through lead body 510 to an epicardial electrode 526. The connector assembly 12 also carries two sets of sealing rings 516 and 520 and a connector ring 518 which, in a unipolar embodiment as illustrated in FIG. 1, is unconnected to conductors within the lead. In the event the invention is practiced within the context of a bipolar embodiment, however, ring 518 would typically be a conductive ring, coupled to an additional conductor located within lead body 510 and extending to a second electrode. An insulative sleeve 522 is provided to ease the insertion of the lead into an implantable pacemaker or other stimulator and to provide strain relief at the point of lead exits the stimulator. At the distal end of the lead is an electrode assembly 524 which carries an epicardial electrode 526, which as illustrated is a steroid eluting epicardial electrode, but which may instead correspond to any of the numerous known epicardial or myocardial pacing electrodes or defibrillation electrodes, including those illustrated in U.S. Patent No. issued to 5,443,492, issued to Stokes, U.S. Patent No. 3,754,344 issued to Bolduc, U.S. Patent No. 5,397,343 issued to Smits, U.S. Patent No. 4,428,818 issued to Doring et al, U.S. Patent No. 5,105,826 issued to Smits et al. and U.S. Patent No. 4,817,634 issued to Holleman et al. Two suture grooves 528 are provided to assist suturing of the electrode head 524 to heart tissue.

In the lead as illustrated, a stranded conductor is provided, located within the coiled conductor connecting electrode 526 to pin 524. The stranded conductor extends from point A, within connector pin 514, to point B, proximal to the distal end of the lead body. In the context of epicardial leads, experience has shown that fractures most commonly occur within this portion of the lead. However, in the context of an endocardial lead, the point of attachment B may be elsewhere along the lead body. Alternatively, in some embodiments, the pacing electrode at the distal end of the lead may itself serve as the point of attachment of the stranded conductor to the coiled conductor.

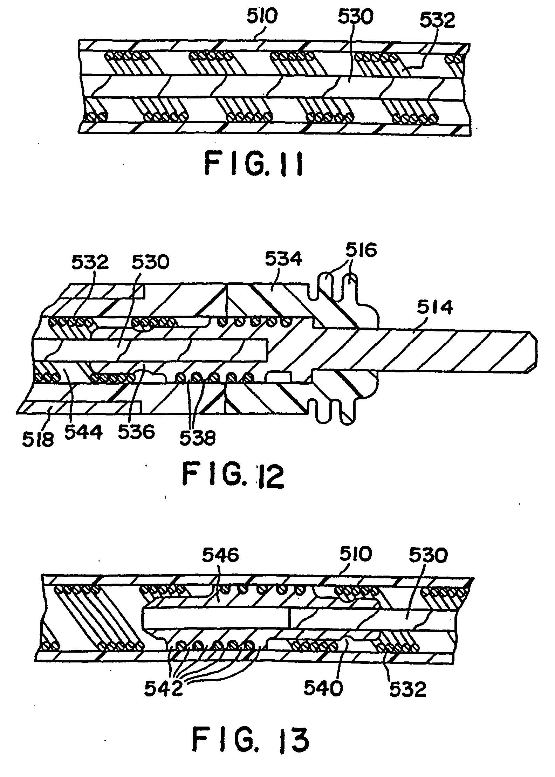

Figure 11 illustrates a sectional view through the lead of Figure 10, at a point intermediate the connector assembly and point B. In this view, stranded conductor 530 and coiled conductor 532 are visible, located within the insulative lead body 510. Coiled conductor 532 is illustrated as a multifilar coil, having five individual filars. However, the present invention may also be used in practice in conjunction with monofilar coils or multifilar coils having different numbers of filars. The stranded conductor 530 preferably takes the form of a cable conductor as described in U.S. Patent No. 5,584,873 issued to Shoberg et al. or a bundled stranded wire conductor as disclosed in U.S. Patent No. 5,246,014, issued to Williams et al. However, stranded or cabled conductors as disclosed in U.S. Patent No. 4964414 issued to Handa, may also be substituted.

Figure 12 illustrates one mechanism for interconnecting the coiled conductor 532, the stranded conductor 530 and the connector pin 514 in the connector assembly of the lead illustrated in Figure 10. Connector pin 514 is fabricated of stainless steel or other biocompatible metal and is provided with an internal lumen in which stranded conductor 530 has been inserted. Stranded connector 530 is retained within connector pin 514 by means of crimps 536. The distal portion of connector pin 514 is provided with external threads 538, in to which individual filars of conductor 532 are screwed. The filars of coil 532 are welded to the adjacent threads 538 on connector pin 514 to provide electrical and mechanical interconnection. Although not illustrated, volume 44 may be backfilled with medical adhesive or other resilient material to provide for stabilization of the connector assembly and for strain relief adjacent the distal end of connector pin 514. Alternatively, coiled conductor 532 may be crimped directly to stranded conductor 530 by means of a cylindrical crimping sleeve surrounding coiled conductor 532 and compressing it against stranded conductor 530. Ring 518 is also visible in cross-section, as are sealing rings 516, molded as part of an insulative polymeric sleeve 34, which is preferably fabricated of silicone rubber.

Figure 13 illustrates the interconnection of the stranded conductor 530 to the coiled conductor 532 at point B, illustrated in Figure 10. A conductive crimp/weld core 546, which may also be fabricated of stainless steel is provided with an internal lumen in which the distal end of stranded conductor 530 is located. Crimps 544 maintain stranded conductor 30 in mechanical and electrical engagement with core 546. Core 546 is also provided with external threads 532, corresponding to threads 538 in FIG. 3, and the individual filars of conductor 532 are welded to the adjacent threads 542 in the manner discussed above in connection with Figure 22 to provide electrical and mechanical interconnection. Alternatively, coiled conductor 532 may be crimped directly to stranded conductor 530 by means of a cylindrical crimping sleeve surrounding coiled conductor 532 and compressing it against stranded conductor 530. In the event that it is desired that the point of interconnection of the coiled conductor 532 and the stranded conductor 530 be located at the distal, pacing electrode, core 546 may take the form of the proximal portion of the electrode, whereby stranded conductor 30 may extend the entire length of the lead.

While Figures 12 and 13 show one method of interconnection of stranded conductor 530 with coiled conductor 532, other mechanisms for connecting these types of conductors, known to the art, may also be substituted. For example, rather than welding conductor 532 to the core or connector pin, it may be engaged on the outer surface of the connector pin or core by means of a circumferential crimping sleeve. Similarly, rather than crimping stranded conductor 530 within core 546 or pin 514, it might instead be crimped within a welding sleeve, and the welding sleeve thereafter welded to either the connector pin or the core. Any interconnection mechanism is believed suitable as long as it provides electrical and mechanical interconnection.

It should be understood that the invention may also usefully be practiced in embodiments having only a single lumen and in embodiments in which multiple lumens are provided by means of single lumen tubes located within larger diameter single lumen tubes.

## Claims

1. An implantable medical lead comprising an elongated body having an elongated lumen therein;
an elongated coiled conductor (116) mounted within the lumen of the lead body;
a multi-strand, generally straight stranded conductor (118), extending along the length of the lead body and coupled electrically to the coiled conductor at a first location and a second location distal to and spaced from the first location; and wherein said coiled conductor (116) defines an internal lumen and wherein said stranded conductor (118) is located within said lumen of said coiled conductor for a distance, along the length of the lead body,
**characterised in that** the stranded conductor (118) and the coiled conductor (116) are electrically and mechanically coupled to one another at said first and second locations.

2. A lead according to claim 1 wherein said lead is provided with an electrical connector (36) located at a proximal end of the lead body and in which the coiled conductor (116) is connected to the connector.

3. A lead according to claim 2 in which the stranded conductor (118) and the coiled conductor (116) are electrically and mechanically coupled to the connector.

4. A lead according to claim 1 provided with an electrical component (16) located on said lead body, wherein said coiled conductor (116) is coupled to said electrical component.

5. A lead according to claim 4 wherein said coiled conductor (116) and said stranded conductor (118) are electrically and mechanically coupled to said electrical component.

6. A lead according to claim 4 or 5 wherein said electrical component is an electrode (16).

7. A lead according to claim 1 wherein the stranded conductor (118) is coupled to the coiled conductor (116) at a point proximal to the electrode (16).

8. A lead according to any preceding claim wherein said stranded conductor (118) is insulated from said coiled conductor (116) between said first and second locations.

9. A lead according to any preceding claim wherein said stranded conductor (118) comprises a seven stranded conductor.

10. A lead according to claim 9 wherein said stranded conductor has an outer diameter of 0.075mm (.003 inches) .

11. A lead according to any preceding claim wherein the stranded conductor (118) and the coiled conductor (116) are electrically insulated from one another along a portion of the coiled conductor intermediate said first and second locations by means of an insulative tubular sheath.

12. A lead according to claim 11 wherein the coiled conductor defines an internal lumen and the tubular sheath is located within the internal lumen of the coiled conductor and the stranded conductor is located within the tubular sheath.

13. A lead according.to any preceding claim wherein said coiled conductor (116) is rotatably mounted in said lumen.

14. A lead according to claim 13 wherein said lead comprises means for coupling said stranded conductor so said coiled conductor while said coiled conductor rotates.

15. A lead according to claim 13 or 14 wherein said lead comprises a rotatably mounted electrical connector located on a proximal portion of said lead body and coupled to said coiled conductor.

16. A lead according to claim 15 wherein said lead comprises a rotatably mounted electrode means located on a distal portion of said electrode body and coupled to said coiled conductor.

## Patentansprüche

1. Implantierbare medizinische Leitung mit:
einem länglichen Körper, der in seinem Innern ein längliches Lumen aufweist;
einem länglichen gewickelten Leiter (116), der im Lumen des Leitungskörpers befestigt ist;
einer mehrsträngigen, im wesentlichen geraden Litze (118), die entlang dem Leitungskörper verläuft und an einer ersten und einer distal und in Abstand von dieser befindlichen zweiten Stelle elektrisch mit dem gewickelten Leiter gekoppelt ist, wobei der gewickelte Leiter (116) ein inneres Lumen definiert und die Litze (118) innerhalb des Lumens des gewickelten Leiters über eine Strecke entlang dem Leitungskörper angeordnet ist,
**dadurch gekennzeichnet, daß** die Litze (118) und der gewickelte Leiter (116) an der ersten und der zweiten Stelle elektrisch und mechanisch miteinander gekoppelt sind.

2. Leitung nach Anspruch 1, wobei die Leitung mit einem elektrischen Anschluß (36) versehen ist, der an einem proximalen Ende des Leitungskörpers angeordnet und mit dem gewikkelten Leiter (116) verbunden ist.

3. Leitung nach Anspruch 2, wobei die Litze (118) und der gewickelte Leiter (116) elektrisch und mechanisch an den Anschluß gekoppelt sind.

4. Leitung nach Anspruch 1, die mit einem an dem Leitungskörper angeordneten elektrischen Bauteil (16) versehen ist, wobei der gewickelte Leiter (116) mit dem elektrischen Bauteil gekoppelt ist.

5. Leitung nach Anspruch 4, wobei der gewickelte Leiter (116) und die Litze (118) elektrisch und mechanisch an das elektrische Bauteil gekoppelt sind.

6. Leitung nach Anspruch 4 oder 5, wobei das elektrische Bauteil eine Elektrode (16) ist.

7. Leitung nach Anspruch 1, wobei die Litze (118) an einem proximal zu der Elektrode (16) gelegenen Punkt mit dem gewikkelten Leiter (116) gekoppelt ist.

8. Leitung nach einem der vorhergehenden Ansprüche, wobei die Litze (118) zwischen der ersten und der zweiten Stelle gegen den gewickelten Leiter (116) isoliert ist.

9. Leitung nach einem der vorhergehenden Ansprüche, wobei die Litze einen siebensträngigen Leiter umfaßt.

10. Leitung nach Anspruch 9, wobei der gewickelte Leiter (118) einen Außendurchmesser von etwa 0,075 mm (0,003 Zoll) aufweist.

11. Leitung nach einem der vorhergehenden Ansprüche, wobei die Litze (118) und der gewickelte Leiter (116) entlang einem Abschnitt des gewickelten Leiters zwischen der ersten und der zweiten Stelle mittels einer rohrförmigen isolierenden Hülle elektrisch gegeneinander isoliert sind.

12. Leitung nach Anspruch 11, wobei der gewickelte Leiter ein inneres Lumen definiert, und die rohrförmige Hülle innerhalb des inneren Lumens des gewickelten Leiters und die Litze innerhalb der rohrförmigen Hülle angeordnet ist.

13. Leitung nach einem der vorhergehenden Ansprüche, wobei der gewickelte Leiter (116) drehbar in dem Lumen befestigt ist.

14. Leitung nach Anspruch 13, wobei die Leitung eine Einrichtung umfaßt, um die Litze mit dem gewickelten Leiter zu koppeln, während sich der gewickelte Leiter dreht.

15. Leitung nach Anspruch 13 oder 14, wobei die Leitung einen drehbar gelagerten elektrischen Anschluß umfaßt, der in einem proximalen Abschnitt des Leitungskörpers angeordnet und mit dem gewickelten Leiter gekoppelt ist.

16. Leitung nach Anspruch 15, wobei die Leitung eine drehbar gelagerte Elektrode umfaßt, die an einem distalen Abschnitt des Elektrodenkörpers angeordnet und mit dem gewickelten Leiter gekoppelt ist.

## Revendications

1. Fil médical implantable comprenant un corps allongé comprenant une lumière allongée ; un conducteur spiralé allongé (116) monté à l'intérieur de la lumière du corps de fil ; un conducteur torsadé (118) en général rectiligne, à plusieurs brins, s'étendant suivant la longueur du corps de fil et couplé électriquement au conducteur spiralé à un premier emplacement et à un deuxième emplacement distal au, et espacé du, premier emplacement ; et dans lequel ledit conducteur spiralé (116) définit une lumière interne et dans lequel ledit conducteur torsadé (118) est placé à l'intérieur de ladite lumière dudit conducteur spiralé à une certaine distance, suivant la longueur dudit corps de fil, **caractérisé en ce que** le conducteur torsadé (118) et le conducteur spiralé (116) sont électriquement et mécaniquement couplés l'un à l'autre au niveau desdits premier et deuxième emplacements.

2. Fil selon la revendication 1, dans lequel ledit fil est muni d'un connecteur électrique (36) placé à une extrémité proximale du corps de fil et dans lequel le conducteur spiralé (116) est connecté au connecteur (36).

3. Fil selon la revendication 2, dans lequel le conducteur torsadé (118) et le conducteur spiralé (116) sont électriquement et mécaniquement couplés au connecteur.

4. Fil selon la revendication 1 muni d'un composant électrique (16) placé sur ledit corps de fil, dans lequel ledit conducteur spiralé (116) est couplé audit composant électrique.

5. Fil selon la revendication 4, dans lequel ledit conducteur spiralé (116) et ledit conducteur torsadé (118) sont électriquement et mécaniquement couplés audit composant électrique.

6. Fil selon la revendication 4 ou 5, dans lequel ledit composant électrique est une électrode (16).

7. Fil selon la revendication 1, dans lequel le conducteur torsadé (118) est couplé au conducteur spiralé (116) à un point proximal de l'électrode (16).

8. Fil selon l'une quelconque des revendications précédentes, dans lequel ledit conducteur torsadé (118) est isolé dudit conducteur spiralé (116) entre lesdits premier et deuxième emplacements.

9. Fil selon l'une quelconque des revendications précédentes, dans lequel ledit conducteur torsadé (116) comprend un conducteur à sept brins.

10. Fil selon la revendication 9, dans lequel ledit conducteur torsadé a un diamètre extérieur de 0,075 mm (0,003 pouce).

11. Fil selon l'une quelconque des revendications précédentes, dans lequel le conducteur torsadé (118) et le conducteur spiralé (116) sont électriquement isolés l'un à l'autre suivant une partie du conducteur spiralé entre lesdits premier et deuxième emplacements au moyen d'une gaine tubulaire isolante.

12. Fil selon la revendication 11, dans lequel le conducteur spiralé définit une lumière interne et la gaine tubulaire est placée à l'intérieur de la lumière interne du conducteur spiralé et le conducteur torsadé est placé à l'intérieur de la gaine tubulaire.

13. Fil selon l'une quelconque des revendications précédentes, dans lequel ledit conducteur spiralé (116) est monté de manière à tourner dans ladite lumière.

14. Fil selon la revendication 13, dans lequel ledit fil comprend des moyens pour coupler ledit conducteur torsadé ( 118) audit conducteur spiralé ( 116) tandis que ledit conducteur spiralé tourne.

15. Fil selon la revendication 13 ou 14, dans lequel ledit fil comprend un connecteur électrique monté de manière à tourner placé sur une partie proximale dudit corps de fil et couplé audit conducteur spiralé.

16. Fil selon la revendication 15, dans lequel ledit fil comprend des moyens d'électrode montés de manière à tourner placés sur une partie distale dudit corps d'électrode et couplés audit conducteur spiralé.
